# EUROPÄISCHE PATENTSCHRIFT

(11) **EP 1 296 655 B1**
(45) Veröffentlichungstag und Bekanntmachung des Hinweises auf die Patenterteilung: **16.07.2008**
(21) Anmeldenummer: 01957856.6
(22) Anmeldetag: 18.06.2001
(51) Int. Cl.: A61K 9/20

(54) **ENDOPARASITIZIDE MITTEL ZUR FREIWILLIGEN ORALEN AUFNAHME DURCH TIERE**
ENDOPARASITICIDAL AGENTS FOR VOLUNTARY ORAL INGESTION BY ANIMALS
AGENTS ENDOPARASITICIDES POUR ABSORPTION ORALE VOLONTAIRE PAR DES ANIMAUX

(30) Priorität: 26.06.2000 DE 10031044
(43) Veröffentlichungstag der Anmeldung: 02.04.2003
(73) Patentinhaber: Bayer HealthCare AG, 51368 Leverkusen (DE)
(72) Erfinder: KALBE, Jochen, 42799 Leichlingen (DE); GEISSLER, Kornelia, 50672 Köln (DE); TRÄUBEL, Michael, 50733 Köln (DE); HARDER, Achim, 51109 Köln (DE); VON SAMSON-HIMMELSTJERNA, Georg, 30900 Mellendorf (DE)
(86) Internationale Anmeldenummer: PCT/EP2001/006836
(87) Internationale Veröffentlichungsnummer: WO 2002/000202

(56) Entgegenhaltungen:
- DE-A- 4 233 625
- DE-A- 4 401 389
- DE-A- 19 853 729
- FR-A- 2 702 960
- FR-A- 2 751 848

## Beschreibung

Die vorliegende Erfindung betrifft oral applizierbare Arzneiformen für Tiere, die von diesen (z.B. von Hunden, Katzen und Pferden) freiwillig aufgenommen werden.

Allgemein üblich werden zur oralen Applikation von Arzneistoffen auch bei Tieren vorzugsweise Tabletten, das heißt Wirk- und Hilfsstoff-Komprimate, verwendet. Diese sind für die Tiere ohne jede Attraktivität und werden von ihnen in der Regel nur unfreiwillig aufgenommen, so dass der Tierhalter die Tabletten in Futter verpacken muss, um sie zu applizieren. Hierbei ist nicht immer garantiert, dass die Arznei vollständig und damit in der richtigen Dosierung appliziert werden kann.

Die Palatabilität dieser Tabletten lässt sich z. B durch Zugabe verschiedener Aromen und Geschmacksstoffe steigern (DE A 196 17 487, WO 95/31963, US 4 851 226). Zusätzlich kann die Form der Tablette geändert werden, wie z.B. als Knochenform bei der Anwendung für Hunde (US 4 857 333). Weiterhin werden Manteltabletten hergestellt, die als äußere Hülle die Attraktivität steigernde Substanzen enthalten (EP A 320 320, EP A 574 301). Der prinzipielle Nachteil dieser verbesserten Tablettensysteme ist die für das Tier offensichtliche Unterscheidbarkeit zum normalen Futter, so dass auch hier keine vollständige Akzeptanz erzielt werden kann.

Für Anwendungen beim Menschen ist die Schmelzextrusion von geeigneten oral applizierbaren Polymeren zu Tabletten bekannt, die aber aufgrund ihrer Konsistenz von den Tieren nicht ausreichend akzeptiert werden (WO 96/29053).

Durch Extrusion von Stärke lassen sich bekanntermaßen verschiedenste Arten von Formkörpern herstellen, die insbesonders in der Futtermittelindustrie Anwendung finden (US 3 899 607). Diese Futtermittel eignen sich jedoch nur bedingt als Träger für pharmazeutische Wirkstoffe, da sie bis zu 50 % mit Fleisch versetzt sind und hierdurch nicht den Regeln einer pharmazeutischen Arzneiform entsprechen. Durch den Fleischzusatz und die entsprechende Form erzielen diese Extrudate jedoch sehr gute Akzeptanz.

Reine Stärkeextrudate mit pharmazeutischen Wirkstoffen (EP A 0 118 240, EP A 390 960) können dagegen keine Akzeptanz erzielen. Die Attraktivität von Futterextrudaten hängt primär von der Aromatisierung aber auch entscheidend von der physikalischen Beschaffenheit ab [M. Thomas et al, Animal Feed Science Technology 70 (1998) 59-78].

Um dem Tierhalter eine möglichst einfache Applikation endoparasitizider Wirkstoffe zu ermöglichen, ist es daher wünschenswert, ein vom Tier freiwillig aufzunehmendes Mittel zu Verfügung zu stellen.

Überraschenderweise wurden nun auf Stärke basierende, extrudierte Formkörper als Arzneiform gefunden, die als Träger für pharmazeutische Wirkstoffe dienen und ohne Fleischzusatz sind, aber von den Tieren freiwillig aufgenommen werden.

Weiterhin Gegenstand der vorliegenden Erfindung ist die Verwendung dieser Arzneiform als Träger für pharmzeutische Wirkstoffe der Veterinärmedizin, inbesondere für cyclische Depsipeptide mit endoparasitizider Wirkung, wie sie z.B. in EP-OS 382 173 und DE-A 4 317 432.9; DE-A 4 317 457.4; DE-A 4 317 458.2 beschrieben sind.

Gegenstand der vorliegenden Erfindung sind:
1. Auf Stärke basierende extrudierte Formkörper, dadurch gekennzeichnet, dass sie spezielle Aromen, Konsistenzgeber und pharmazeutische Arzneistoffe für Tiere beinhalten, erhältlich, in dem man die Einsatzstoffe mischt und bei Temperaturen unter 150°C weiterverarbeitet.
2. Auf Stärke basierende extrudierte Formkörper gemäß Punkt 1, dadurch gekennzeichnet, dass sie als Aromen Geflügelleberaroma oder Fleischaroma enthalten.
3. Auf Stärke basierende extrudierte Formkörper gemäß Punkt 1, dadurch gekennzeichnet, dass sie eine Shore-A-Härte von 10 bis 100 aufweisen.
4. Auf Stärke basierende extrudierte Formkörper gemäß Punkt 1 und 2, dadurch gekennzeichnet, dass sie cyclische Depsipeptide, bestehend aus Aminosäuren und Hydroxycarbonsäuren als Bausteinen und mit 6 bis 30 Ring- oder Kettenatomen, enthalten.
5. Auf Stärke basierende extrudierte Formkörper gemäß Punkt 1, 2 und 3, dadurch gekennzeichnet, dass sie mit pulverförmigen Celluloseacetat versetzt sind.
6. Auf Stärke basierende extrudierte Formkörper gemäß Punkt 1, 2, 3 und 4, dadurch gekennzeichnet, dass sie weitere Hilfsstoffe wie Emulgatoren, Feuchthaltemittel und Konservierungsstoffe enthalten.
7. Verfahren zu Herstellung von auf Stärke basierenden extrudierten Formkörpern gemäß Punkt 1, 2, 3, 4 und 5 dadurch gekennzeichnet, dass die Einsatzstoffe gemischt werden und bei Temperaturen unter 150°C weiterverarbeitet werden.

Als Wirkstoffe kommen prinzipiell alle für den Einsatz in der Veterinärmedizin geeigneten Wirkstoffe in Frage. Besonders geeignet sind die Wirkstoffe aus der Klasse der Depsipeptide, insbesondere cyclische Depsipeptide.

Bevorzugte cyclische Depsipeptide sind solche mit 18 bis 24 Ringatomen, insbesondere mit 24 Ringatomen.

Zu den Depsipeptiden mit 18 Ringatomen zählen Verbindungen der allgemeinen Formel (I): 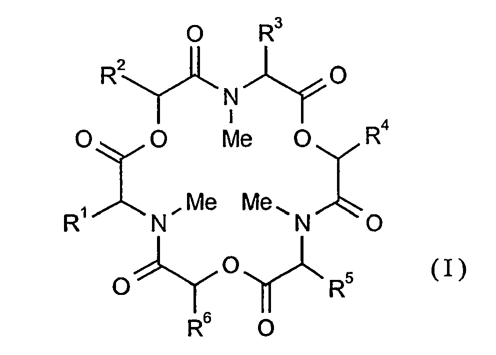 in welcher
- R¹, R³ und R⁵: unabhängig voneinander für Wasserstoff, geradkettiges oder verzweigtes Alkyl mit bis zu 8 Kohlenstoffatomen, Hydroxyalkyl, Alkanoyloxyalkyl, Alkoxyalkyl, Aryloxyalkyl, Mercaptoalkyl, Alkylthioalkyl, Alkylsulfinylalkyl, Alkylsulfonylalkyl, Carboxyalkyl, Alkoxycarbonylalkyl, Arylalkoxycarbonylalkyl, Carbamoylalkyl, Aminoalkyl, Alkylaminoalkyl, Dialkylaminoalkyl, Guanidinoalkyl, das gegebenenfalls durch einen oder zwei Benzyloxycarbonylreste oder durch einen, zwei, drei oder vier Alkylreste substituiert sein kann, Alkoxycarbonylaminoalkyl, 9-Fluorenylmethoxycarbonyl(Fmoc)aminoalkyl, Alkenyl, Cycloalkyl, Cycloalkylalkyl sowie gegebenenfalls substituiertes Arylalkyl, wobei als Substituenten genannt seien Halogen, Hydroxy, Alkyl und Alkoxy, stehen,
- R², R⁴ und R⁶: unabhängig voneinander für Wasserstoff, geradkettiges oder verzweigtes Alkyl mit bis zu 8 Kohlenstoffatomen, Hydroxyalkyl, Mercaptoalkyl, Alkanoyloxyalkyl, Alkoxyalkyl, Aryloxyalkyl, Alkylthioalkyl, Alkylsulfinylalkyl, Alkylsulfonylalkyl, Carboxyalkyl, Alkoxycarbonylalkyl, Arylalkoxycarbonylalkyl, Carbamoylalkyl, Aminoalkyl, Alkylaminoalkyl, Dialkylaminoalkyl, Alkoxycarbonylaminoalkyl, Alkenyl, Cycloalkyl, Cycloalkylalkyl, gegebenenfalls substituiertes Aryl oder Arylalkyl wobei als Substituenten genannt seien Halogen, Hydroxy, Alkyl, Alkoxy, stehen,
sowie deren optische Isomere und Racemate.

Bevorzugt sind Verbindungen der Formel (I), 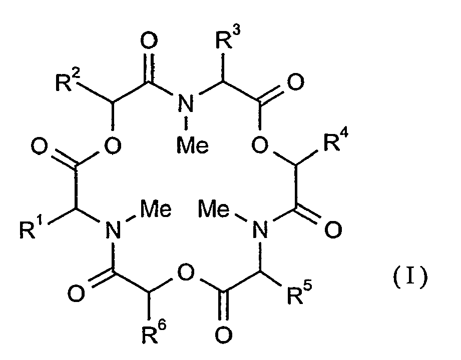 in welcher
- R¹, R³ und R⁵: unabhängig voneinander für geradkettiges oder verzweigtes C₁-C₈-Alkyl, insbesondere Methyl, Ethyl, Propyl, Isopropyl, Butyl, Isobutyl, sec.-Butyl, tert.- Butyl, Pentyl, Isopentyl, sec.-Pentyl, Hexyl, Isohexyl, sec.-Hexyl, Heptyl, Isoheptyl, sec.-Heptyl, tert.-Heptyl, Oktyl, Isooktyl, sec.-Oktyl, Hydroxy-C₁-C₆-alkyl, insbesondere Hydroxymethyl, 1-Hydroxyethyl, C₁-C₄-Alkanoyloxy-C₁-C₆-alkyl, insbesondere Acetoxymethyl, 1-Acetoxyethyl, C₁-C₄-Alkoxy-C₁-C₆-alkyl, insbesondere Methoxymethyl, 1-Methoxyethyl, Aryl-C₁-C₄-alkyloxy-C₁-C₆-alkyl, insbesondere Benzyloxymethyl, 1-Benzyloxy-ethyl, Mercapto-C₁-C₆-alkyl, insbesondere Mercaptomethyl, C₁-C₄-Alkylthio-C₁-C₆-alkyl, insbesondere Methylthioethyl, C₁-C₄-Alkylsulfinyl-C₁-C₆-alkyl, insbesondere Methylsulfinylethyl, C₁-C₄-Akylsulfonyl-C₁-C₆-alkyl, insbesondere Methylsulfonylethyl, Carboxy-C₁-C₆-alkyl, insbesondere Carboxymethyl, Carboxyethyl, C₁-C₄-Alkoxycarbonyl-C₁-C₆-alkyl, insbesondere Methoxycarbonylmethyl, Ethoxycarbonylethyl, C₁-C₄-Arylalkoxycarbonyl-C₁-C₆-alkyl, insbesondere Benzyloxycarbonyhnethyl, Carbamoyl-C₁-C₆-alkyl, insbesondere Carbamoylmethyl, Carbamoylethyl, Amino-C₁-C₆-alkyl, insbesondere Aminopropyl, Aminobutyl, C₁-C₄-Alkylamino-C₁-C₆-alkyl, insbesondere Methylaminopropyl, Methylaminobutyl, C₁-C₄-Dialkylamino-C₁-C₆-alkyl, insbesondere Dimethylaminopropyl, Dimethylaminobutyl, Guanido-C₁-C₆-alkyl, insbesondere Guanidopropyl, C₁-C₄-Alkoxycarbonylamino-C₁-C₆-alkyl, insbesondere tert.-Butoxycarbonylaminopropyl, tert.-Butoxycarbonylaminobutyl, 9-Fluorenylmethoxycarbonyl(Fmoc)amino-C₁-C₆-alkyl, insbesondere 9-Fluorenyl-methoxycarbonyl(Fmoc)aminopropyl, 9-Fluorenylmethoxycarbonyl-(Fmoc)aminobutyl, C₂-C₈-Alkenyl, insbesondere Vinyl, Allyl, Butenyl, C₃-C₇-Cycloalkyl, insbesondere Cyclopentyl, Cyclohexyl, Cycloheptyl, C₃-C₇-Cycloalkyl-C₁-C₄-alkyl, insbesondere Cyclopentylmethyl, Cyclohexylmethyl, Cycloheptylmethyl, Phenyl-C₁-C₄-alkyl, insbesondere Phenylmethyl das gegebenenfalls durch Reste aus der Reihe Halogen, insbesondere Fluor, Chlor Brom oder Iod, Hydroxy, C₁-C₄- Alkoxy, insbesondere Methoxy oder Ethoxy und C₁-C₄-Alkyl, insbesondere Methyl, substituiert sein kann, stehen
- R², R⁴ und R⁶: unabhängig voneinander für geradkettiges oder verzweigtes C₁-C₈-Alkyl, insbesondere Methyl, Ethyl, Propyl, Isopropyl, Butyl, Isobutyl, sec.-Butyl, tert.-Butyl, Pentyl, Isopentyl, sec.-Pentyl, Hexyl, Isohexyl, sec.-Hexyl, Heptyl, Isoheptyl, sec.-Heptyl, tert.-Heptyl, Oktyl, Isooktyl, sec.-Oktyl, Hydroxy-C₁-C₆-alkyl, insbesondere Hydroxymethyl, 1-Hydroxyethyl, C₁-C₄-Alkanoyloxy-C₁-C₆-alkyl, insbesondere Acetoxymethyl, 1-Acetoxyethyl, C₁-C₄-Alkoxy-C₁-C₆-alkyl, insbesondere Methoxymethyl, 1-Methoxyethyl, Aryl-C₁-C₄-alkyloxy-C₁-C₆-alkyl, insbesondere Benzyloxymethyl, 1-Benzyloxy-ethyl, Mercapto-C₁-C₆-alkyl, insbesondere Mercaptomethyl, C₁-C₄-Alkylthio-C₁-C₆-alkyl, insbesondere Methylthioethyl, C₁-C₄-Alkylsulrinyl-C₁-C₆-alkyl, insbesondere Methylsulfinylethyl, C₁-C₄-Alkylsulfonyl-C₁-C₆-alkyl, insbesondere Methylsulfonylethyl, Carboxy-C₁-C₆-alkyl, insbesondere Carboxymethyl, Carboxyethyl, C₁-C₄-Alkoxycarbonyl-C₁-C₆-alkyl, insbesondere Methoxycarbonylmethyl, Ethoxycarbonylethyl, C₁-C₄-Arylalkoxycarbonyl-C₁-C₆-alkyl, insbesondere Benzyloxycarbonylmethyl, Carbamoyl-C₁-C₆-alkyl, insbesondere Carbamoylmethyl, Carbamoylethyl, Amino-C₁-C₆-alkyl, insbesondere Aminopropyl, Aminobutyl, C₁-C₄-Alkylamino-C₁-C₆-alkyl, insbesondere Methylaminopropyl, Methylaminobutyl, C₁-C₄-Dialkylamino-C₁-C₆-alkyl, insbesondere Dimethylaminopropyl, Dimethylaminobutyl, C₂-C₈-Alkenyl, insbesondere Vinyl, Allyl, Butenyl, C₃-C₇-Cycloalkyl, insbesondere Cyclopentyl, Cyclohexyl, Cycloheptyl, C₃-C₇-Cyctoalkyl-C₁-C₄-alkyl, insbesondere Cyclopentylmethyl, Cyclohexylmethyl, Cycloheptylmethyl, Phenyl, Phenyl-C₁-C₄-alkyl, insbesondere Phenylmethyl das gegebenenfalls durch Reste aus der Reihe Halogen, insbesondere Fluor, Chlor, Brom oder Iod, Hydroxy, C₁-C₄-Alkoxy, insbesondere Methoxy oder Ethoxy und C₁-C₄-Alkyl, insbesondere Methyl, substituiert sein kann, stehen sowie deren optische Isomere und Racemate.

Besonders bevorzugt sind Verbindungen der Formel (I),
in welcher
- R¹, R³ und R⁵: unabhängig voneinander für geradkettiges oder verzweigtes C₁-C₈-Alkyl, insbesondere Methyl, Ethyl, Propyl, Isopropyl, Butyl, Isobutyl, sec.-Butyl, Pentyl, Isopentyl, sec.-Pentyl, Hexyl, Isohexyl, sec.-Hexyl, Heptyl, Isoheptyl, sec.-Heptyl, Oktyl, Isooktyl, sec.-Oktyl, Hydroxy-C₁-C₆-alkyl, insbesondere Hydroxymethyl, 1-Hydroxyethyl, C₁-C₄-Alkanoyloxy-C₁-C₆-alkyl, insbesondere Acetoxymethyl, 1-Acetoxyethyl, C₁-C₄-Alkoxy-C₁-C₆-alkyl, insbesondere Methoxymethyl, 1-Methoxyethyl, Aryl-C₁-C₄-alkyloxy-C₁-C₆-alkyl, insbesondere Benzyloxymethyl, 1-Benzyloxyethyl, C₁-C₄-Alkoxycarbonylamino-C₁-C₆-alkyl, insbesondere tert.-Butoxycarbonylaminopropyl, tert.-Butoxycarbonylaminobutyl, C₂-C₈-Alkenyl, insbesondere Vinyl, Allyl, C₃-C₇-Cycloalkyl, insbesondere Cyclopentyl, Cyclohexyl, Cycloheptyl, C₃-C₇-Cycloalkyl-C₁-C₄-alkyl, insbesondere Cyclopentylmethyl, Cyclohexylmethyl, Cycloheptylmethyl, Phenyl-C₁-C₄-alkyl, insbesondere Phenylmethyl das gegebenenfalls durch einen oder mehrere gleiche oder verschiedene der oben angegebenen Reste substituiert sein kann, stehen
- R², R⁴ und R⁶: unabhängig voneinander für geradkettiges oder verzweigtes C₁-C₈-Alkyl, insbesondere Methyl, Ethyl, Propyl, Isopropyl, Butyl, Isobutyl, sec.-Butyl, tert.-Butyl, Pentyl, Isopentyl, sec.-Pentyl, Hexyl, Isohexyl, sec.-Hexyl, Heptyl, Isoheptyl, sec.-Heptyl, tert.-Heptyl, Oktyl, Isooktyl, sec.-Oktyl, Hydroxy-C₁-C₆-alkyl, insbesondere Hydroxymethyl, Aryl-C₁-C₄-alkyloxy-C₁-C₆-alkyl, insbesondere Benzyloxymethyl, 1-Benzyloxyethyl, Carboxy-C₁-C₆-alkyl, insbesondere Carboxymethyl, Carboxyethyl, C₁-C₄-Alkoxycarbonyl-C₁-C₆-alkyl, insbesondere Methoxycarbonylmethyl, Ethoxycarbonylethyl, C₁-C₄-Aryl-alkoxycarbonyl-C₁-C₆-alkyl, insbesondere Benzyloxycarbonyhnethyl, C₁-C₄-Alkylmnino-C₁-C₆-alkyl, insbesondere Methylaminopropyl, Methylaminobutyl, C₁-C₄-Dialkylamino-C₁-C₆-alkyl, insbesondere Dimethylaminopropyl, Dimethylaminobutyl, C₂-C₈-Alkenyl, insbesondere Vinyl, Allyl, Butenyl, C₃-C₇-Cycloalkyl, insbesondere Cyclopentyl, Cyclohexyl, Cycloheptyl, C₃-C₇-Cycloalkyl-C₁-C₄-alkyl, insbesondere Cyclopentylmethyl, Cyclohexylmethyl, Cycloheptyl-methyl, Phenyl, Phenyl-C₁-C₄-alkyl, insbesondere Phenylmethyl das gegebenenfalls durch einen oder mehrere gleiche oder verschiedene der oben angegebenen Reste substituiert sein kann, stehen sowie deren optische Isomere und Racemate.

Ganz besonders bevorzugt sind Verbindungen der Formel (I),
in welcher
- R¹, R³ und R⁵: unabhängig voneinander für geradkettiges oder verzweigtes C₁-C₈-Alkyl, insbesondere Methyl, Ethyl, Propyl, Isopropyl, Butyl, Isobutyl, sec.-Butyl, Pentyl, Isopentyl, sec.-Pentyl, Hexyl, Isohexyl, sec.-Hexyl, Heptyl, Isoheptyl, sec.-Heptyl, Oktyl, Isooktyl, sec.-Oktyl, C₂-C₈-Alkenyl, insbesondere Allyl, C₃-C₇-Cycloalkyl-C₁-C₄-alkyl, insbesondere Cyclohexylmethyl, Phenyl-C₁-C₄-alkyl, insbesondere Phenylmethyl, stehen
- R², R⁴ und R⁶: unabhängig voneinander für geradkettiges oder verzweigtes C₁-C₈-Alkyl, insbesondere Methyl, Ethyl, Propyl, Isopropyl, Butyl, Isobutyl, sec.-Butyl, Pentyl, Isopentyl, sec.-Pentyl, Hexyl, Isohexyl, sec.-Hexyl, Heptyl, Isoheptyl, sec.-Heptyl, Oktyl, Isooktyl, sec.-Oktyl, C₂-C₈- Alkenyl, insbesondere Vinyl, Allyl, C₃-C₇-Cycloalkyl-C₁-C₄-alkyl, insbesondere Cyclohexylmethyl, Phenyl-C₁-C₄-alkyl, insbesondere Phenylmethyl das gegebenenfalls durch einen oder mehrere gleiche oder verschiedene der oben angegebenen Reste substituiert sein kann, stehen
sowie deren optische Isomere und Racemate.

Im Sinne der vorliegenden Erfindung können alle Verbindungen der allgemeinen Formel (I), die in optisch aktiven, stereoisomeren Formen oder als racemische Gemische vorliegen können, verwendet werden. Vorzugsweise werden jedoch die optisch aktiven, stereoisomeren Formen der Verbindungen der allgemeinen Formel (I) erfindungsgemäß verwendet.

Im Einzelnen seien folgende Verbindungen der allgemeinen Formel (I) genannt, in welcher
die Reste R¹ bis R⁶ die folgende Bedeutung haben:

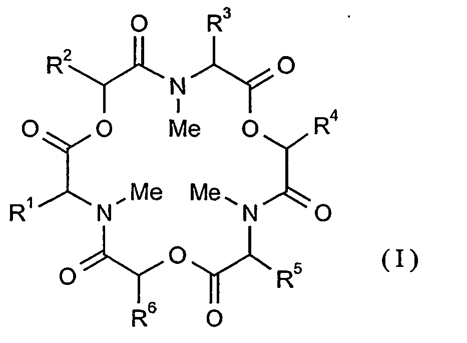

| **R¹** | **R²** | **R³** | **R⁴** | **R⁵** | **R⁶** |
|---|---|---|---|---|---|
| -CHMeCH₂Me | -Cyclohexyl | -CHMeCH₂Me | -Me | -CEMeCH₂Me | -Me |
| -CHMeCH₂Me | -Cyclohexyl | -CHMeCH₂Me | -Me | -CHMeCH₂Me | -Cyclohexyl |
| -CHMeCH₂Me | -CH₂-Phe | -CHMeCH₂Me | -Me | -CHMeCH₂Me | -Me |
| -CHMeCH₂Me | -CH₂-Phe | -CHMeCH₂Me | -Me | -CHMeCH₂Me | -CH₂-Phe |
| -CHMeCH₂Me | -(CH₂)₃-Me | -CEMeCH₂Me | -Me | -CHMeCH₂Me | -Me |
| -CHMeCH₂Me | -(CH₂)₃-Me | -CHMeCH₂Me | -Me | -CHMeCH₂Me | -(CH₂)₃-Me |
| -CHMe₂ | -CH₂-Phe | -CHMeCH₂Me | -Me | -CHMeCH₂Me | -Me |
| -CH₂-Phe | -CHMe₂ | -CH₂-Phe | -CHMe₂ | -CHMeCH₂Me | -CHMe₂ |
| -CH₂CHMe₂ | -CH₂-Phe | -CH₂CHMe₂ | -Me | -CH₂CHMe₂ | -CH₂-Phe |
| -(CH₂)₃-Me | -Me | -CHMeCH₂Me | -Me | -CHMeCH₂Me | -Me |
| -CHMe₂ | -Me | -CHMe₂ | -Me | -CHMe₂ | -Me |
| -CH₂-Me | -Me | -CH₂-Me | -Me | -CH₂-Me | -Me |
| -(CH₂)₂-Me | -Me | -(CH₂)₂-Me | -Me | -(CH₂)₂-Me | -Me |
| -(CH₂)₃-Me | -Me | -(CH₂)₃-Me | -Me | -(CH₂)₃-Me | -Me |
| -CH₂-CH=CH₂ | -Me | -CH₂-CH=CH₂ | -Me | -(CH₂)-CH=CH₂ | -Me |
| -CHMeCH₂Me | -Me | -CHMeCH₂Me | -Me | -CHMeCH₂Me | -CH₂-Me |
| -CHMeCH₂Me | -Me | -CHMeCH₂Me | -Me | -CHMeCH₂Me | -(CH₂)₂-Me |
| -CHMeCH₂Me | -Me | -CHMeCH₂Me | -Me | -CHMeCH₂Me | -(CH₂)₃-Me |
| -CHMeCH₂Me | -Me | -CHMeCH₂Me | -Me | -CH₂Me | -Me |
| -CHMeCH₂Me | -Me | -CHMeCH₂Me | -Me | -(CH₂)₂-Me | -Me |
| -Cyclohexyl | -Me | -Cyclohexyl | -Me | -Cyclohexyl | -Me |
| -CH₂CHMe₂ | -Cyclohexyl | -CH₂CHMe₂ | -Me | -CH₂CHMe₂ | -Cyclohexyl |
| -CH₂CHMe₂ | -Cyclohexyl | -CH₂CEMe₂ | -Me | -CH₂CHMe₂ | -Me |
| -CHMeCH₂Me | -CHMe₂ | -CHMeCH₂Me | -CHMe₂ | -CHMeCH₂Me | -Me |
| -CH₂-Phe | -Me | -CH₂-Phe | -Me | -CH₂-Phe | -Me |
| -Cyclohexyl | -Me | -Cyclohexyl | -Me | -Cyclohexyl | -Me |
| -CHMe₂ | -CHMe₂ | -CHMe | -Me | -CHMe₂ | -Me |
| -CHMe₂ | -CHMe₂ | -CHMe₂ | -CHMe₂ | -CHMe₂ | -Me |
| -CH₂-Me | -CHMe₂ | -CH₂Me | -Me | -CH₂-Me | -Me |
| -CH₂-Me | -CHMe₂ | -CHMe₂ | -CHMe₂ | -CH₂-Me | -Me |
| -(CH₂)₂-Me | -CHMe₂ | -(CH₂)₂-Me | -Me | -(CH₂)₂-Me | -Me |
| -(CH₂)₂-Me | -CHMe₂ | -(CH₂)₂-Me | -CHMe₂ | -(CH₂)₂-Me | -Me |
| -(CH₂)₃-Me | -CHMe₂ | -(CH₂)₃-Me | -Me | -(CH₂)₃-Me | -Me |
| -(CH₂)₃-Me | -CHMe₂ | -(CH₂)₃-Me | -CHMe₂ | -(CH₂)₃-Me | -Me |
| -CH₂-CH=CH₂ | -CHMe₂ | -CH₂-CH=CH₂ | -Me | -CH₂-CH=CH₂ | -Me |
| -CH₂-CH=CH₂ | -CHMe₂ | -CH₂-CH=CH₂ | -CHMe₂ | -CH₂-CH=CH₂ | -Me |
| -Me | -Me | -CHMeCH₂Me | -Me | -CH₂-Me | -Me |
| -Me | -Me | -CHMeCH₂Me | -Me | -(CH₂)₃-Me | -Me |

| | | | | | |
|---|---|---|---|---|---|
| Me = Methyl; Phe = Phenyl | | | | | |

Weiterhin sei als Depsipeptid die aus EP-OS 382 173 bekannte Verbindung PF 1022 der folgenden Formel genannt:

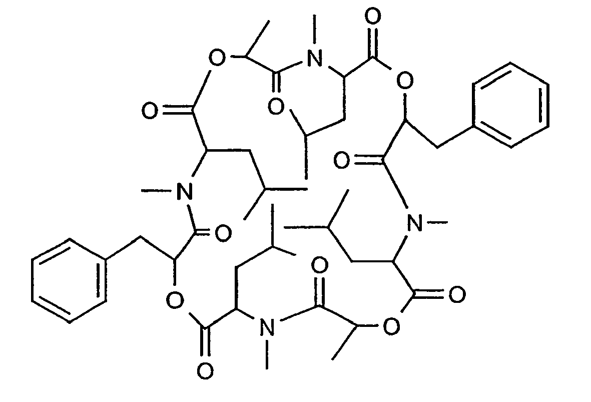

Außerdem seien als Depsipeptide die aus der PCT-Anmeldung WO 93/19053 bekannten Verbindungen genannt.

Insbesondere seien aus PCT-Anmeldung WO 93/19053 die Verbindungen der folgenden Formel genannt: 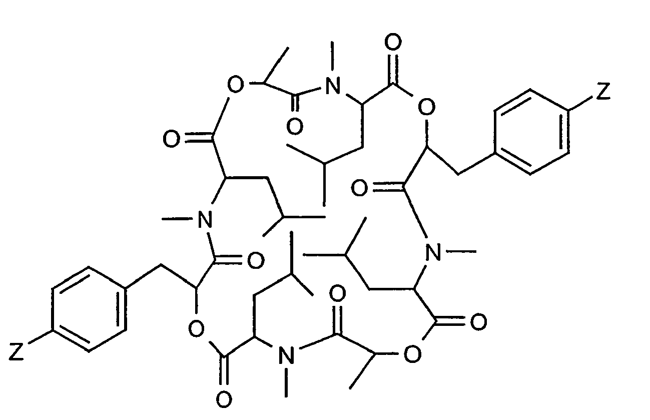 in welcher
- Z: für N-Morpholinyl, Amino, Mono- oder Dimethylamino steht.

Ein besonders bevorzugtes Beispiel für diese Verbindungen ist das Bis-morpholino-Derivat Cyclo[D-2-hydroxypropanoyl-N-methyl-L-leucyl-3-[4-(4-morpholinyl)-phenyl]-D-2-hydroxypropanoyl-N-methyl-L-leucyl-D-2-hydroxypropanoyl-N-methyl-L-leucyl-3[4-(4-morpholinyl)phenyl]-D-2-hydroxypropanoyl-N-methyl-L-leucyl] (CAS 155030-63-0).

Außerdem seien Verbindungen der folgenden Formel genannt: 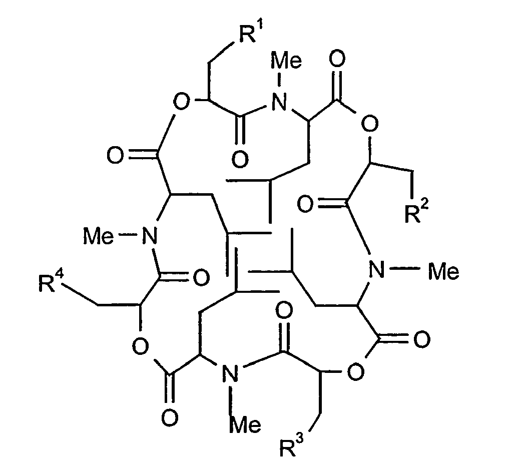 in welcher
- R¹, R², R³, R⁴: unabhängig voneinander für Wasserstoff, C₁-C₁₀-Alkyl oder Aryl, insbesondere Phenyl stehen, die gegebenenfalls substituiert sind durch Hydroxy, C₁-C₁₀-Alkoxy oder Halogen.

Die Verbindungen der allgemeinen Formel (I) sind bekannt und können nach den in EP-A-382 173, DE-A 4 317 432, DE-A 4 317 457, DE-A 4 317 458, EP-A-634 408, EP-A-718 293, EP-A-872 481, EP-A-685 469, EP-A-626 375, EP-A-664 297, EP-A-669343, EP-A-787 141, EP-A-865498, EP-A-903 347 beschriebenen Verfahren erhalten werden.

Zu den cyclischen Depsipeptiden mit 24 Ringatomen zählen auch Verbindungen der allgemeinen Formel (Ia) 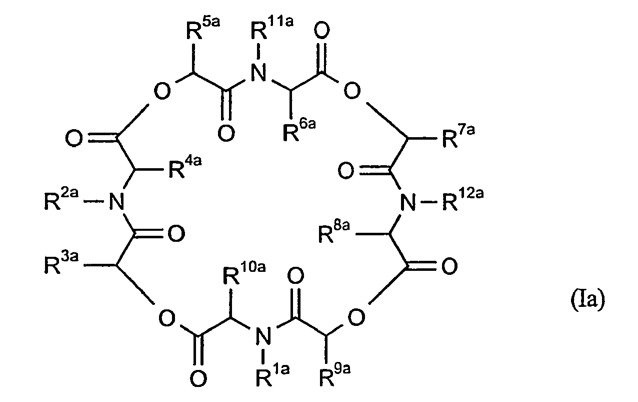 in welcher
- R^{1a}, R^{2a}, R^{11a} und R^{12a}: unabhängig voneinander für C₁₋₈-Alkyl, C₁₋₈-Halogenalkyl, C₃₋₆-Cycloalkyl, Aralkyl, Aryl stehen,
- R^{3a}, R^{5a}, R^{7a}, R^{9a}: unabhängig voneinander für Wasserstoff oder geradkettiges oder verzweigtes C₁₋₈-Alkyl stehen, das gegebenenfalls durch Hydroxy, C₁₋₄-Alkoxy, Carboxy, 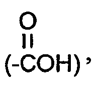 Carboxamid, 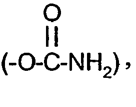 Imidazolyl, Indolyl, Guanidino, -SH oder C₁₋₄-Alkylthio substituiert sein kann und ferner für Aryl oder Aralkyl die durch Halogen, Hydroxy, C₁₋₄-Alkyl, C₁₋₄-Alkoxy substituiert sein können, stehen,
- R^{4a}, R^{6a}, R^{8a}, R^{10a}: unabhängig voneinander für Wasserstoff, geradkettiges C₁₋₅-Alkyl, C₂₋₆-Alkenyl, C₃₋₇-Cycloalkyl, die gegebenenfalls durch Hydroxy, C₁₋₄-Alkoxy, Carboxy, Carboxamid, Imidazolyl, Indolyl, Guanidino, SH oder C₁₋₄-Alkylthio substituiert sein können, sowie für Aryl oder Aralkyl die durch Halogen, Hydroxy, C₁₋₄-Alkyl, C₁₋₄-Alkoxy substituiert sein können, stehen
sowie deren optische Isomere und Racemate.

Bevorzugt werden Verbindungen der Formel (Ia) eingesetzt, in welcher
- R^{1a}, R^{2a}, R^{11a} und R^{12a}: unabhängig voneinander für Methyl, Ethyl, Propyl, Isopropyl, n-, s-, t-Butyl oder Phenyl, das gegebenenfalls substituiert ist durch Halogen, C₁₋₄-Alkyl, OH, C₁₋₄-Alkoxy, sowie für Benzyl oder Phenylethyl stehen, die gegebenenfalls durch die bei Phenyl angegebenen Reste substituiert sein können;
- R^{3a} bis R^{10a}: die oben angegebene Bedeutung haben.

Besonders bevorzugt sind Verbindungen der Formel (Ia), in welcher
- R^{1a}, R^{2a}, R^{11a} und R^{12a}: unabhängig voneinander für Methyl, Ethyl, Propyl, Isopropyl oder n-, s-, t-Butyl stehen,
- R^{3a}, R^{5a}, R^{7a}, R^{9a}: für Wasserstoff, geradkettiges oder verzweigtes C₁₋₈-Alkyl, insbesondere Methyl, Ethyl, Propyl, i-Propyl, n-, s-, t-Butyl, die gegebenenfalls durch C₁₋₄-Alkoxy, insbesondere Methoxy, Ethoxy, Imidazolyl, Indolyl oder C₁₋₄-Alkylthio, insbesondere Methylthio, Ethylthio substituiert sein können, ferner für Phenyl, Benzyl oder Phenethyl stehen, die gegebenenfalls durch Halogen, insbesondere Chlor, substituiert sein können.
- R^{4a}, R^{6a}, R^{8a}, R^{10a}: unabhängig voneinander für Wasserstoff, Methyl, Ethyl, n-Propyl, n-Butyl, Vinyl, Cyclohexyl, die gegebenenfalls durch Methoxy, Ethoxy, Imidazolyl, Indolyl, Methylthio, Ethylthio substituiert sein können sowie für Isopropyl, s-Butyl ferner für jeweils gegebenenfalls halogensubstituiertes Phenyl, Benzyl oder Phenylethyl stehen.

Die Verbindungen der Formel (Ia) können ebenfalls nach den in EP-A-382 173, DE-A 4 317 432, DE-A 4 317 457, DE-A 4 317 458, EP-A-634 408, EP-A-718 293, EP-A-872 481, EP-A-685 469, EP-A-626 375, EP-A-664 297, EP-A-669 343, EP-A-787 141, EP-A-865 498, EP-A-903 347 beschriebenen Verfahren erhalten werden.

Die erfindungsgemäßen Mittel eignen sich bei günstiger Warmblütertoxizität zur Bekämpfung von pathogenen Endoparasiten die bei Menschen und in der Tierhaltung und Tierzucht bei Nutz-, Zucht-, Zoo-, Labor-, Versuchs- und Hobbytieren vorkommen. Sie sind dabei gegen alle oder einzelne Entwicklungsstadien der Schädlinge sowie gegen resistente und normal sensible Arten wirksam. Durch die Bekämpfung der pathogenen Endoparasiten sollen Krankheit, Todesfälle und Leistungsminderungen (z.B. bei der Produktion von Fleisch, Milch, Wolle, Häuten, Eiern, Honig usw.) vermindert werden, so dass durch den Einsatz der Wirkstoffe eine wirtschaftlichere und einfachere Tierhaltung möglich ist. Zu den pathogenen Endoparasiten zählen Cestoden, Trematoden, Nematoden, Acantocephalen insbesondere:

Aus der Ordnung der Pseudophyllidea z.B.: Diphyllobothrium spp., Spirometra spp., Schistocephalus spp., Ligula spp., Bothridium spp., Diphlogonoporus spp.

Aus der Ordnung der Cyclophyllidea z.B.: Mesocestoides spp., Anoplocephala spp., Paranoplocephala spp., Moniezia spp., Thysanosomsa spp., Thysaniezia spp., Avitellina spp., Stilesia spp., Cittotaenia spp., Andyra spp., Bertiella spp., Taenia spp., Echinococcus spp., Hydatigera spp., Davainea spp., Raillietina spp., Hymenolepis spp., Echinolepis spp., Echinocotyle spp., Diorchis spp., Dipylidium spp., Joyeuxiella spp., Diplopylidium spp.

Aus der Unterklasse der Monogenea z.B.: Gyrodactylus spp., Dactylogyrus spp., Polystoma spp.

Aus der Unterklasse der Digenea z.B.: Diplostomum spp., Posthodiplostomum spp., Schistosoma spp., Trichobilharzia spp., Ornithobilharzia spp., Austrobilharzia spp., Gigantobilharzia spp., Leucochloridium spp., Brachylaima spp., Echinostoma spp., Echinoparyphium spp., Echinochasmus spp., Hypoderaeum spp., Fasciola spp., Fasciolides spp., Fasciolopsis spp., Cyclocoelum spp., Typhlocoelum spp., Paramphistomum spp., Calicophoron spp-, Cotylophoron spp., Gigantocotyle spp., Fischoederius spp., Gastrothylacus spp., Notocotylus spp., Catatropis spp., Plagiorchis spp., Prosthogonimus spp., Dicrocoelium spp., Eurytrema spp., Troglotrema spp., Paragonimus spp., Collyriclum spp., Nanophyetus spp., Opisthorchis spp., Clonorchis spp. Metorchis spp., Heterophyes spp., Metagonimus spp.

Aus der Ordnung der Enoplida z.B.: Trichuris spp., Capillaria spp., Trichomosoides spp., Trichinella spp.

Aus der Ordnung der Rhabditia z.B.: Micronema spp., Strongyloides spp.

Aus der Ordnung der Strongylida z.B.: Stronylus spp., Triodontophorus spp., Oesophagodontus spp., Trichonema spp., Gyalocephalus spp., Cylindropharynx spp., Poteriostomum spp., Cyclococercus spp., Cylicostephanus spp., Oesophagostomum spp., Chabertia spp., Stephanurus spp., Ancylostoma spp., Uncinaria spp., Bunostomum spp.,

Globocephalus spp., Syngamus spp., Cyathostoma spp., Metastrongylus spp., Dictyocaulus spp., Muellerius spp., protostrongylus spp., Neostrongylus spp., Cystocaulus spp., Pneumostrongylus spp., Spicocaulus spp., Elaphostrongylus spp. Parelaphostrongylus spp., Crenosoma spp., Paracrenosoma spp., Angiostrongylus spp., Aelurostrongylus spp., Filaroides spp., Parafilaroides spp., Trichostrongylus spp., Haemonchus spp., Ostertagia spp., Marshallagia spp., Cooperia spp., Nematodirus spp., Hyostrongylus spp., Obeliscoides spp., Amidostomum spp., Ollulanus spp.

Aus der Ordnung der Oxyurida z.B.: Oxyuris spp., Enterobius spp., Passalurus spp., Syphacia spp., Aspiculuris spp., Heterakis spp.

Aus der Ordnung der Ascaridia z.B.: Ascaris spp., Toxascaris spp., Toxocara spp., Parascaris spp., Anisakis spp., Ascaridia spp.

Aus der Ordnung der Spirurida z.B.: Gnathostoma spp., Physaloptera spp., Thelazia spp., Gongylonema spp., Habronema spp., Parabronema spp., Draschia spp., Dracunculus spp.

Aus der Ordnung der Filariida z.B.: Stephanofilaria spp., Parafilaria spp., Setaria spp., Loa spp., Dirofilaria spp., Litomosoides spp., Brugia spp., Wuchereria spp., Onchocerca spp.

Aus der Ordnung der Gigantorhynchida z.B.: Filicollis spp., Moniliformis spp., Macracanthorhynchus spp., Prosthenorchis spp.

Zu den Nutz- und Zuchttieren gehören Säugetiere wie z.B. Rinder, Pferde, Schafe, Schweine, Ziegen, Kamele, Wasserbüffel, Esel, Kaninchen, Damwild, Rentiere, Pelztiere wie z.B. Nerze, Chinchilla, Waschbär, Vögel wie z.B. Hühner, Gänse, Puten und Enten, Strauße, Süß- und Salzwasserfische wie z.B. Forellen, Lachse, Karpfen und/oder Aale, Reptilien, sowie Insekten wie z.B. Honigbiene und Seidenraupe.

Zu Labor- und Versuchstieren gehören Mäuse, Ratten, Meerschweinchen, Goldhamster, Hunde und Katzen.

Zu den Hobbytieren gehören Hunde und Katzen.
Die erfindungsgemäßen Mittel werden besonders bevorzugt bei Hunden und Katzen, insbesondere Hunden eingesetzt.

Die Anwendung kann sowohl prophylaktisch als auch therapeutisch erfolgen.

Die erfindungsgemäßen Formkörper können auch als Träger für die Verabreichung anderer Wirkstoffe verwendet werden. Als Beispiele seien genannt: Andere Wirkstoffe die gegen pathogene Endoparasiten wirken wie z.B. L-2,3,5,6-Tetrahydro-6-phenylimidazothiazol, Benzimidazolcarbamate wie Febantel, ferner Pyrantel, Praziquantel und Ivermectin; Coccidiostatika, wie Toltrazuril und Ponazuril (=Toltrazuril-Sulfon); Schmerzmittel wie Flupirtin und Antibiotika wie Enrofloxacin und die in WO 97/31001 beschriebenen Verbindungen, insbesondere 8-Cyan-1-cyclopropyl-7-((1S,6S)-2,8-diazabicyclo[4.3.0]nonan-8-yl)-6-fluor-1,4-dihydro-4-oxo-3-chinolincarbonsäure der Formel

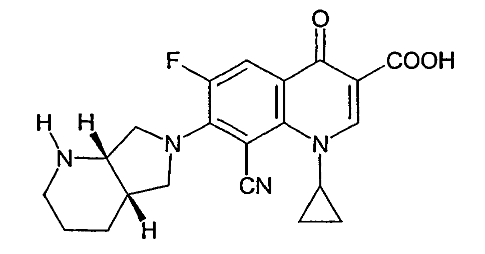

Die Wirkstoffe können in den erfindungsgemäßen Formkörpern auch in Kombination mit Synergisten oder mit anderen geeigneten Wirkstoffen eingesetzt werden. Beispielsweise können die weiter oben angeführten Depsipeptide mit anderen Wirkstoffen gegen pathogene Endoparasiten , wie sie z. B weiter oben genannt sind, kombiniert werden.

Anwendungsfertige Zubereitungen enthalten die Wirkstoffe in Konzentrationen von 10 ppm - 25 Gewichtsprozent, bevorzugt von 0,1-20 Gewichtsprozent.

Im allgemeinen hat es sich als vorteilhaft erwiesen, Mengen der erfindungsgemäßen Mischung von etwa 0,001 bis etwa 100 mg Wirkstoff je kg Körpergewicht pro Tag zur Erzielung wirksamer Ergebnisse zu verabreichen. Bevorzugt sind 0,005 bis 5 mg Wirkstoff je kg Körpergewicht.

Als Hilfsstoffe werden verwendet: Stärke wie beispielsweise Stärke aus Weizen, Reis, Mais, Tapioca, Roggen, Hafer und Kartoffeln. Modifizierte Stärken können physikalisch vorbehandelte Stärken wie vorgekochte oder chemisch veränderte wie Hydroxyethylstärke, Hydroxypropylstärke, Methylstärke, Carboxymethylstärke, Stärkeacetat, Hydroxypropylstärkeacetat, Hydroxyethylstärkeacetat, Stärkephosphate, Stärkesulfate, oder chemisch oder ionisch vernetzte Stärken wie Distärkephosphate, Phosphate hydroxypropylierter Stärken, Stärkedicarbonsäurediester oder Salze anionischer Stärkederivate sein. Bevorzugt sind hydroxypropylierte und phosphatvernetzte Stärken von Mais, Weizen, Tapioka und Kartoffel. Hierbei werden Stärkemengen zwischen 30% und 80% bevorzugt zwischen 40% und 70% und besonders bevorzugt zwischen 40 und 60 % eingesetzt. Die Prozentangaben sind dabei Gewichtsprozent des fertigen Mittels.

Weiterhin werden Zucker wie Saccharose, Glucose, Fructose, Mannose und Sorbit verwendet. Hierbei werden Mengen zwischen 1% und 20%, bevorzugt zwischen 1% und 15% und besonders bevorzugt zwischen 1% und 10% eingesetzt. Die Prozentangaben sind dabei Gewichtsprozent des fertigen Mittels.

Zur Form- und Konsistenzgebung eignen sich besonders Cellulose und deren Derivate wie mikrokristalline Cellulose, Hydroxypropylcellulose, Methylhydroxypropylcellulose, Carboxymethylcellulose besonders Celluloseacetat und ganz besonders Cellulose-2,5-Acetat. Weiterhin geeignet sind hochdisperse Silicate und Titandioxid. Hierbei werden Mengen zwischen 1% und 40%, bevorzugt zwischen 1 % und 30% und besonders bevorzugt zwischen 1 und 20% eingesetzt. Die Prozentangaben sind dabei Gewichtsprozent des fertigen Mittels.

Als Feuchthaltemittel und Weichmacher dienen Wasser, Glycerin, Propylenglykol, Polyethylenglykole und Polypropylenglykole. Hierbei werden Mengen zwischen 1% und 30%, bevorzugt zwischen 5% und 30% und besonders bevorzugt zwischen 5 und 20% eingesetzt. Die Prozentangaben sind dabei Gewichtsprozent des fertigen Mittels.

Als Konservierungsstoffe können die für pharmazeutische Präparate und Lebensmittel üblichen Verbindungen wie Benzoesäureester, p-Hydroxybenzoesäuremethylester, p-Hydroxybenzoesäurethylester, p-Hydroxybenzoesäurepropylester, Sorbinsäure, Propylgallat, Citronensäure, Ascorbinsäure, Ascorbinpalmitat, Tocopherol, Tocopherolacetat, Butylhydroxytoluol und Butylhydroxyanisol eingesetzt werden.

Als Emulgatoren können eingesetzt werden: Tenside wie
1. nichtionogene, z.B. polyoxyethyliertes Rizinusöl, polyoxyethoxyliertes Sorbitan-Monooleat, Sorbitan-Monostearat, Ethylalkohol, Glycerinmonostearat, Polyoxyethylstearat, Alkylphenolpolylglykolether,
2. ampholytische, wie Di-Na-N-lauryl-β-iminodipropionat oder Lecithin,
3. anionaktive, wie Na-Laurylsulfat, Fettalkoholethersulfate, Mono/Dialkylpolyglykoletherorthophosphorsäureester-Monoethanolaminsalz,

Hierbei betragen die eingesetzten Mengen vorzugsweise von 0,05 Gew.-% bis 2 Gew.-% bezogen auf die Gesamtmenge an Inhaltsstoffen. Besonders bevorzugt sind Mengen von 0,2 bis 1 Gew.-%.

Die erfindungsgemäßen Formkörper weisen idealerweise eine Shore-A-Härte von 10 bis 100, bevorzugt von 10 bis 65, ganz besonders bevorzugt von 10 bis 30 und insbesondere von 15 bis 25 auf. Die Shore-A-Härte wird dabei gemäß DIN Methode 53505 ermittelt.

Als Aromen sind Trockenleberpulver aus Rind, Geflügel, Schaf, oder Schwein bevorzugt aus Geflügel und Schwein, sowie andere Aromenzubereitungen geeignet. Hierbei werden Mengen zwischen 1% und 30%, bevorzugt zwischen 5% und 25% und besonders bevorzugt zwischen 5% und 20% eingesetzt. Die Prozentangaben sind dabei Gewichtsprozent des fertigen Mittels.

Ganz besonders geeignet sind die unter den Bezeichnungen BEEF^{®} und BAYOPAL^{®} im Handel befindlichen Aromen der Firmen Pharmachem (BEEF^{®}) und Haarmann und Reimer (BAYOPAL^{®}).

Die folgenden Beispiele erläutern die Erfindung ohne sie einzuschränken. Als Wirkstoff wird in den Beispielen die Verbindung Cyclo[D-2-hydroxypropanoyl-N-methyl-L-leucyl-3-[4-(4-morpholinyl)phenyl]-D-2-hydroxypropanoyl-N-methyl-L-leucyl-D-2-hydroxypropanoyl-N-methyl-L-leucyl-3[4-(4-morpholinyl)phenyl]-D-2-hydroxypropanoyl-N-methyl-L-leucyl] (CAS 155030-63-0) eingesetzt.

### Beispiel 1

55 % Weizenmehl, 10 %Fructose, 10% Beef-Aroma, Pharma-Chemie, 1 % Aerosil und 4% Depsipeptid werden homogenisiert und gesiebt und anschließend über eine Dosierschnecke dem Extruder hinzugefügt. Über eine Dosierpumpe werden entsprechend 5%Wasser und 15% Glycerin (bezogen auf die Gesamtmischung) hinzugepumpt. Die Extrusionstemperatur beträgt 120°C. Der entstehende Strang wird in Stücke geschnitten, so dass ein Stück die Dosierung für 10 kg Körpergewicht Tier enthält. Die Prozentangaben sind dabei als Gewichtsprozente zu verstehen.

### Beispiel 2

45% Maisstärke, 10% Saccharose, 10% Leber-Aroma, Haarmann& Reimer, 10% Celluloseacetatpulver, 1% Aerosil und 4% Depsipeptid werden homogenisiert und gesiebt und anschließend über eine Dosierschnecke dem Extruder hinzugefügt. Über eine Dosierpumpe werden entsprechend 5% Wasser und 15% Glycerin (bezogen auf die Gesamtmischung) hinzugepumpt. Die Extrusionstemperatur beträgt 120°C. Der entstehende Strang wird in Stücke geschnitten, so dass ein Stück die Dosierung für 10 kg Körpergewicht Tier enthält. Die Prozentangaben sind dabei als Gewichtsprozente zu verstehen.

### Beispiel 3

Die in Beispiel 2 hergestellten Muster werden an Hunde verfüttert. Hierbei werden sowohl Placebo-Muster (ohne Wirkstoff) wie Verum-Muster (mit Wirkstoff) gegen ein kommerziell erhältliches fleischhaltiges Futter ("Frolic") getestet. Die Akzeptanz sowohl der Placebo- als auch der Verum-Muster ist vergleichbar.

### Beispiel 4

Die Muster aus Beispiel 1 oder 2 werden in einer Dosierung von 5 mg Depsipeptid pro kg Körpergewicht an mit Parasiten infizierte Hunde verfüttert. Nach zwei bis vier Tagen sind die Tiere parasitenfrei.

| **Tier** | **Parasit** | **Wirkung** |
|---|---|---|
| 2 Hunde | Toxocara canis | 3/3 |
| 2 Hunde | Ancylostoma caninum | 3/3 |

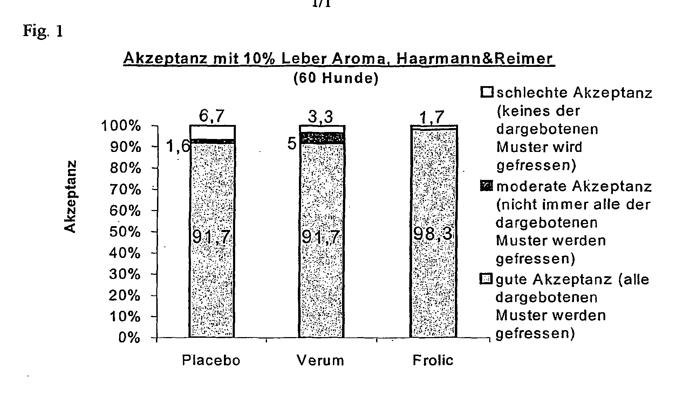

## Patentansprüche

1. Auf Stärke basierende extrudierte Formkörper, **dadurch gekennzeichnet, dass** sie spezielle Aromen, Konsistenzgeber und pharmazeutische Arzneistoffe für Tiere beinhalten, erhältlich, indem man die Einsatzstoffe mischt und bei Temperaturen unter 150°C weiterverarbeitet.

2. Auf Stärke basierende extrudierte Formkörper gemäß Anspruch 1, **dadurch gekennzeichnet, dass** sie als Aromen Geflügelleberaroma oder Fleischaroma enthalten.

3. Auf Stärke basierende extrudierte Formkörper gemäß Anspruch 1, **dadurch gekennzeichnet, dass** sie eine Shore-A- Härte von 10 bis 100 aufweisen.

4. Auf Stärke basierende extrudierte Formkörper gemäß Anspruch 1 und 2, **dadurch gekennzeichnet, dass** sie cyclische Depsipeptide, bestehend aus Aminosäuren und Hydroxycarbonsäuren als Bausteinen und mit 6 bis 30 Ring- oder Kettenatomen, enthalten.

5. Auf Stärke basierende extrudierte Formkörper gemäß Anspruch 1, 2 und 3, **dadurch gekennzeichnet, dass** sie mit pulverförmigen Celluloseacetat versetzt sind.

6. Auf Stärke basierende extrudierte Formkörper gemäß Anspruch 1, 2, 3 und 4, **dadurch gekennzeichnet, dass** sie weitere Hilfsstoffe wie Emulgatoren, Feuchthaltemittel und Konservierungsstoffe enthalten.

7. Verfahren zu Herstellung von auf Stärke basierenden extrudierten Formkörpern gemäß Anspruch 1, 2, 3 und 4, **dadurch gekennzeichnet, dass** die Einsatzstoffe gemischt werden und bei Temperaturen unter 150°C weiterverarbeitet werden.

## Claims

1. Starch-based extruded shaped articles, **characterized in that** they comprise specific aromas, bodying agents and pharmaceutical active compounds for animals, obtainable by mixing the starting materials and processing the mixture at temperatures of less than 150°C.

2. Starch-based extruded shaped articles according to Claim 1, **characterized in that** they contain poultry liver aroma or meat aroma as aromas.

3. Starch-based extruded shaped articles according to Claim 1, **characterized in that** they have a Shore A hardness of 10 to 100.

4. Starch-based extruded shaped articles according to Claims 1 and 2, **characterized in that** they contain cyclic depsipeptides composed of amino acids and hydroxycarboxylic acids as units and having 6 to 30 ring or chain atoms.

5. Starch-based extruded shaped articles according to Claims 1, 2 and 3, **characterized in that** they have added to them pulverulent cellulose acetate.

6. Starch-based extruded shaped articles according to Claims 1, 2, 3 and 4, **characterized in that** they contain further ancilliary materials such as emulsifiers, humectants and preservatives.

7. Process for the preparation of starch-based extruded shaped articles according to Claims 1, 2, 3 and 4, **characterized in that** the starting materials are mixed and processed at temperatures of less than 150°C.

## Revendications

1. Corps façonnés extrudés à base de matière amylacée, **caractérisés en ce qu'**ils comprennent des aromatisants spéciaux, des agents de consistance et des produits pharmaceutiques médicamenteux pour animaux, que l'on obtient en mélangeant les ingrédients et en poursuivant le traitement à des températures au-dessous de 150°C.

2. Corps façonnés extrudés à base de matière amylacée suivant la revendication 1, **caractérisés en ce qu'**ils contiennent comme aromatisants des arômes de foie de volaille ou des arômes de viande.

3. Corps façonnés extrudés à base de matière amylacée suivant la revendication 1, **caractérisés en ce qu'**ils présentent une dureté Shore-A de 10 à 100.

4. Corps façonnés extrudés à base de matière amylacée suivant les revendications 1 et 2, **caractérisés en ce qu'**ils contiennent des depsipeptides cycliques ayant comme éléments constitutifs des acides aminés et des acides hydroxycarboxyliques et comprenant 6 à 30 atomes de carbone cycliques ou en chaîne.

5. Corps façonnés extrudés à base de matière amylacée suivant les revendications 1, 2 et 3, **caractérisés en ce qu'**ils sont additionnés d'acétate de cellulose en poudre.

6. Corps façonnés extrudés à base de matière amylacée suivant les revendications 1, 2, 3 et 4, **caractérisés en ce qu'**ils contiennent d'autres substances auxiliaires telles que des émulsifiants, des agents humectants et des conservateurs.

7. Procédé de production de corps façonnés extrudés à base de matière amylacée suivant les revendications 1, 2, 3 et 4, **caractérisé en ce que** les ingrédients sont mélangés et leur traitement est poursuivi à des températures au-dessous de 150°C.
